(19) **Europäisches Patentamt European Patent Office Office européen des brevets**

(11) **EP 3 496 809 B1**

(12) **EUROPEAN PATENT SPECIFICATION**

(45) Date of publication and mention of the grant of the patent:
**13.08.2025 Bulletin 2025/33**

(21) Application number: **17758953.8**

(22) Date of filing: **31.07.2017**

(51) International Patent Classification (IPC):
**A61N 2/02** *(2006.01)*    **C12M 1/42** *(2006.01)*

(52) Cooperative Patent Classification (CPC):
**A61N 2/02**

(86) International application number:
**PCT/IB2017/054659**

(87) International publication number:
**WO 2018/029569 (15.02.2018 Gazette 2018/07)**

(54) **APPARATUS AND METHOD FOR THE DETERMINATION AND THE APPLICATION OF ELECTROMAGNETIC FIELDS FOR INFLUENCING IN VITRO CELL GROWTH**

**VORRICHTUNG UND VERFAHREN ZUR BESTIMMUNG UND ANWENDUNG VON ELEKTROMAGNETISCHEN FELDERN ZUR BEEINFLUSSUNG VON IN-VITRO-ZELLWACHSTUM**

**APPAREIL ET PROCÉDÉ POUR LA DÉTERMINATION ET L'APPLICATION DE CHAMPS ÉLECTROMAGNÉTIQUES POUR INFLUENCER LA CROISSANCE CELLULAIRE IN VITRO**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.08.2016 IT 201600083775**

(43) Date of publication of application:
**19.06.2019 Bulletin 2019/25**

(73) Proprietor: **Politecnico Di Torino**
**10129 Torino (IT)**

(72) Inventors:
- **LUCIA, Umberto**
  **15121 Alessandria (IT)**
- **MONTRUCCHIO, Bartolomeo**
  **10129 Torino (IT)**
- **GREGORETTI, Francesco**
  **10129 Torino (IT)**
- **PAOLUCCI, Emilio**
  **10129 Torino (IT)**
- **BORCHIELLINI, Romano**
  **10143 Torino (IT)**
- **BRESSAN, Maurizio**
  **10090 San Giusto Canavese (TO) (IT)**
- **PONZETTO, Antonio Emilio Gaspare**
  **10024 Moncalieri (TO) (IT)**
- **SILVAGNO, Francesca**
  **10126 Torino (IT)**
- **GERVINO, Gianpiero Renato**
  **10127 Torino (IT)**

(74) Representative: **Ferroni, Filippo et al**
**Metroconsult Genova S.r.l.**
**Via Palestro, 5/2**
**16122 Genova (IT)**

(56) References cited:
**EP-A1- 0 966 988          WO-A1-2009/153818**
**WO-A1-2013/048546      WO-A2-2005/010162**
**DE-A1- 102010 013 718**

EP 3 496 809 B1

## Description

In a most general aspect thereof, the present invention relates to the application of low-frequency electromagnetic fields on cells, especially human cells or other mammals, although other living organisms may be used.

The use of electromagnetic radiation for influencing the cell growth and/or reproduction is a phenomenon known in the art since the beginning of the last century, when the first radiotherapy trials using ionizing radiation began.

The technological evolution that followed in the '900 and in recent years has led to the creation of modern medical equipment, rather complex and sophisticated; think for example of MRI devices that generate high-frequency alternating magnetic fields, or those for radiotherapy that make use of accelerated electron beams, gamma rays, X rays or the like.

In one case, the devices have coils operated with high frequencies to generate the magnetic fields, while in the other there are radiation sources (e.g. X-ray tubes) or particle accelerators (linear or circular).

Without dwelling further on this type of medical equipment, which is well known and therefore reference should be made to what is amply documented at a scientific and technical level, it should be noted that the application of electromagnetic fields or radiation is not only used for medical purposes, but also in laboratory with machines which are intended to operate on cell or biological tissue samples in vitro.

Irrespective of the medical or laboratory applications mentioned above, one aspect that is common to the various devices that make use of electromagnetic fields is that they are operatively little flexible as they work according to preset work programs.

In practice, all the devices are of the electronic type and are associated with a processor (a computer or the like) or are equipped with electronic components such as processors, storages, connections with devices, control commands and whatever else is necessary to ensure that an operator can set their operation according to a work program.

Thus, for example, in the case of magnetic resonances, in the devices it is possible to determine the intensity of the magnetic field and direct its flow towards the parts of the body to be treated, while in laboratory devices this is done on the specimens arranged inside them.

The duration and/or intensity of radiation or electromagnetic fields are selected as a function of predetermined protocols, so that at the end of the working session, the results achieved (e.g. images, x-rays, tomograms, etc.) concur to form a report which is used for the purposes of the case.

The known apparatuses do not provide the opportunity to adapt their operating parameters to the performance of the work session, namely the results measured in real time.

This is due essentially to the fact that apart from the dosage of the electromagnetic radiation or fields, which depend in practice on the time and intensity initially set on the equipment for each cycle, it is not possible to change other operating parameters in order to adapt them to different situations, in particular during a session.

This condition does not allow for an immediate evaluation, or at least in the short term, of the effects of electromagnetic fields on cell or biological tissue samples; in fact, they are usually analyzed by evaluating the effects of electromagnetic fields deducing them from the variations of the cell mass, but this requires a certain time.

In addition, no teaching exists in the prior art as to how to modify the electromagnetic fields as a function of the effects observed on the analyzed cell samples; it therefore follows that the effectiveness of laboratory equipment that apply electromagnetic fields to in vitro cell samples, is somewhat limited.

Consider for example the case in which one wants to know whether the application of electromagnetic fields on tumor cell lines has reached the desired effects, such as the reduction of proliferation or whether all the areas subjected to the field have been reached, so as to avoid having to repeat it.

In the literature, methods have been proposed to suppress and/or inhibit the growth of cancer cells based on the use of magnetic fields at very low frequency, i.e. frequencies typically in the order of a few Hertz (Hz) or a few tens of Hz, and anyway lower than 1 kHz.

Of these magnetic fields, also known among the experts by the acronym "ELF" ( "Extremely Low Frequency" magnetic field), the effects are known to induce necrosis and/or apoptosis in cancer cells without harming healthy cells, demonstrated especially experimentally in vitro and/or in laboratory animals.

For more information on the subject, please refer to the extensive literature on the subject, of which only a few articles published are cited herein as possible reference examples: "Bioelectromagnetic Field Effects on Cancer Cells and Mice Tumors" [H. Berg, B. Gunther, I. Hilger, M. Radeva, N. Traitcheva, L. Wollber - Electromagnetic Biology and Medicine, 29: 132-143, 2010]; "In Vitro Exposure Apparatus for ELF Magnetic Fields" - J. Schuderer, W. Oesch, N. Felber, D. Spät, N. Kuster - Bioelectro magnetics 25:582-591 (2004)].

Other systems for treatment of cells with magnetic fields are described in publications WO 2013/048546 A1, WO 2005/010162 A2 and WO 2009/153818 A1.

Without wanting to go into that from a scientific standpoint, as this would not be the appropriate place, it is noted that as part of scientific research, laboratory equipment has been developed operating with ELF magnetic fields, an example of which is described in the first one of the articles mentioned above while others can also be found in patent publications.

For the latter, reference can be made to European patent application EP 0 966 988 (on behalf of Tofani) which describes a machine that generates ELF low-frequency magnetic fields, alone or combined with SELF static fields (Static Electromagnetic Field), the intensity of which is of a few mT (1 to 30).

According to the different possible variants shown in this European patent application, the electromagnetic fields are generated with coils which can be arranged coaxially to each other, one inside the other or spaced apart with an interposed work space, so as to concentrate the flow lines in an area where the specimens are placed.

The coils are preferably Helmholtz (i.e. directional type) while the apparatus has an amplifier and a modulator for their power supply, according to treatment cycles set or programmed by an electronic processor (such as a personal computer or the like).

The experimental results of the various treatments are based on the observations of the samples and the cell count with techniques based on the use of microscopes or histological analysis, with a subsequent evaluation of statistical type (e.g. T Student distributions or other types).

Similar considerations also apply with regard to the laboratory equipment described in the article "In Vitro Exposure Apparatus for ELF Magnetic Fields" mentioned above, which focuses essentially only on the structural description of the apparatus, which is capable of providing controlled conditions of the magnetic fields within it, without interest on the treatments to be performed and the evaluation of their effectiveness.

In general, it can be said that the prior art does not provide useful teachings to modify or otherwise control the magnetic fields inside the equipment, in terms of their effects on the detected cell samples.

In the light of this discussion, it can be said that one of the technical problems underlying the present invention is to provide an apparatus for the application of low-frequency magnetic fields on cell samples having structural and operating features such as to overcome the limits outlined with reference to the prior art.

Another technical problem related to the invention is that of devising a method which allows modifying and/or controlling the electromagnetic fields applied to the cell samples as a function of the effects recorded on them.

The idea of solution of this problem is to provide an apparatus adapted to perform applications of electromagnetic fields, preferably alternating and/or pulsed with low frequency ELF, SELF type on in vitro cell samples, which allows correlating the effects on the cells to the magnetic fields applied so as to be able to optionally modify or correct the latter.

**In** particular, the Applicants have been able to observe that an apparatus in which it is possible to modify the frequency of the magnetic fields as a function of at least one parameter such as the variation of the cell size, or the variation of the total cell mass, allows influencing the growth of cells in a predetermined and repeatable manner. Preferably, the apparatus is intended to operate on tumor cells, as the Applicants have surprisingly observed a different effect of the alternating magnetic fields on tumor cells, whose growth and/or reproduction is slowed compared to healthy ones of the fields that are not substantially affected by the above magnetic fields.

The features of the invention are set out more specifically in the following claims; such features will appear more clearly in the light of the following description of a possible embodiment thereof, illustrated only by way of non-limiting example in the accompanying figures, in which:

- Fig. 1 is a general block diagram of an apparatus according to the invention;
- Fig. 2 is a further diagram showing some components of fig. 1 and the connections thereof;
- Fig. 3, 4 and 5 are a front, side and plan view, respectively, of the apparatus in the preceding figures;
- Fig. 6 is a circuit diagram of an amplifier module of the apparatus in the preceding figures;
- Fig. 7 and 8 show respective variants of a structure of the apparatus in fig. 1.

With reference to the listed figures, reference numeral 1 therein generally designates a device for the treatment of tumor cells, according to the invention.

Before proceeding with the detailed description, it is necessary to note that particular configurations and/or structures and/or features described hereinafter with reference to the non-limiting example can be taken individually or combined in any suitable manner, in one or more embodiments, also different from the embodiments exemplified; moreover, the reference numerals used hereinafter are for convenience only and do not define the scope of protection or the scope of the embodiments. The numeral and spatial references and/or definitions such as "upper", "lower", "above", "below", "top", "bottom" refer to the exemplary figures and should not be construed in a limiting sense.

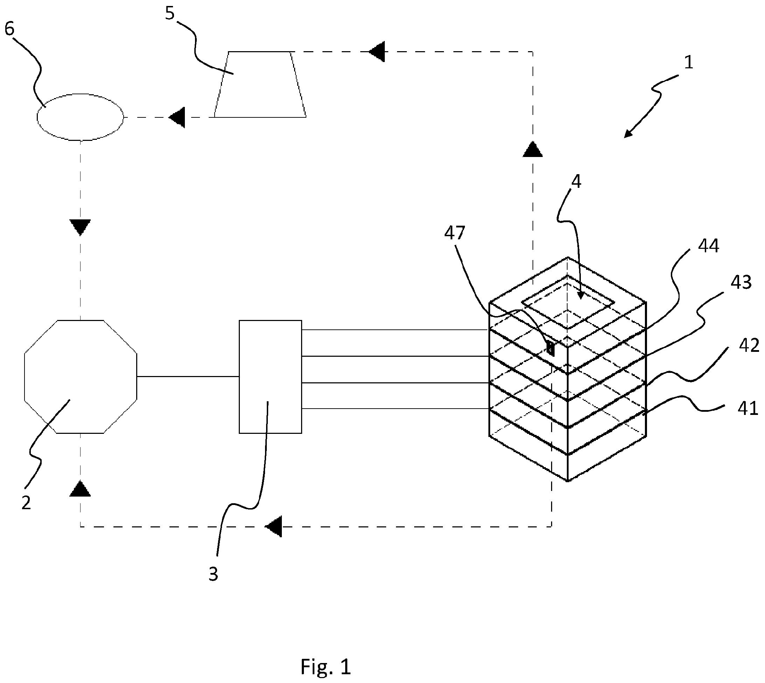

From a logical-functional point of view, apparatus 1 essentially consists of a series of groups or blocks shown in figure 1: a function generator unit 2, a power amplifier unit 3, a magnetic cage or structure 4, a cell feature detection unit 5, a computation unit 6.

The function generator 2 is a device adapted to impart an oscillating signal to the amplifier unit or module 3; to this end, generator 2 is adapted to generate electrical signals of oscillating type and transmit them to amplifier 3 to which it is connected.

According to a preferred embodiment of the invention, the waveform provided by the function generator 2 is modulated in amplitude and/or frequency and/or in shape, depending on the characteristics of the tumor cells to be treated, such as chemical, physical, biological features.

In this context, it should be noted that the use of sine and square waves has been found to be particularly effective, while the frequencies used are preferably comprised in a range of from a few Hz (including fractions such as 0.2-0.5 Hz) up to a few tens (90-100 Hz); to this end, commercially available function generators may be used, or custom-built by assembling components that can be purchased on the market.

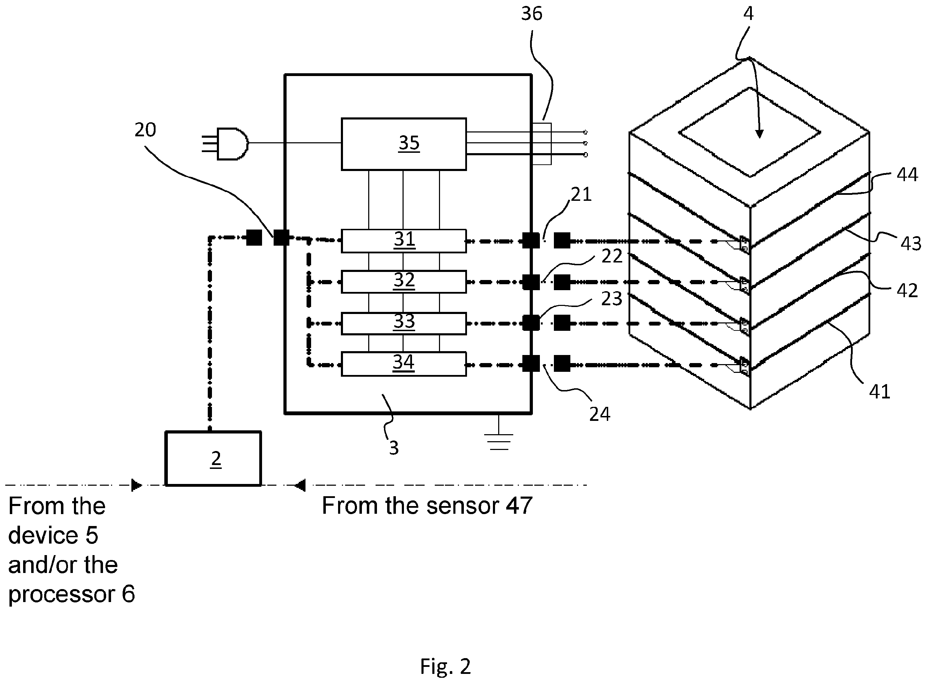

The function generator 2 is connected to the amplifier unit 3, better shown in fig. 2 from which it is possible to see that it comprises a series of modules 31, 32, 33, 34, as many as are the coils 41, 42, 43, 44 of the magnetic structure 4 to be fed.

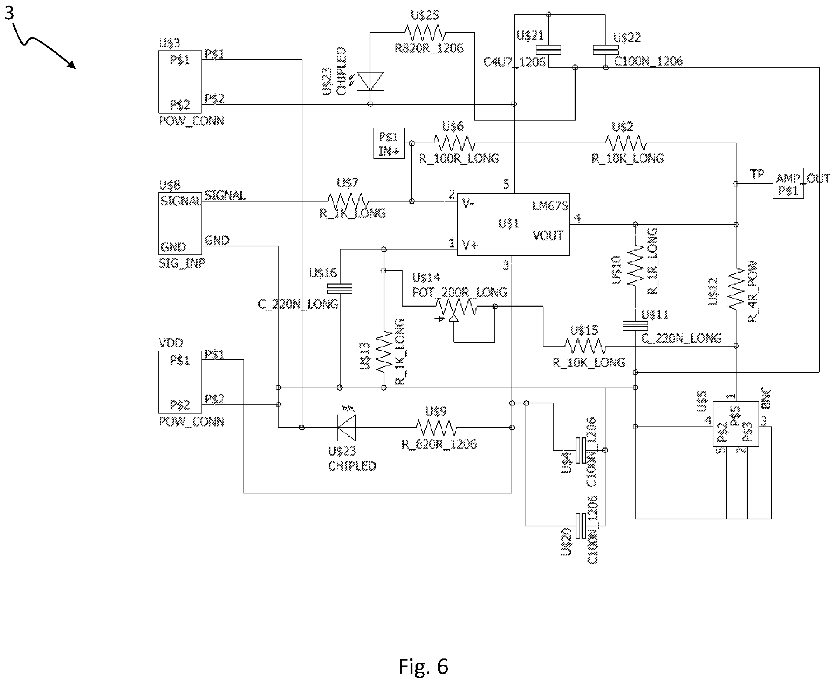

Modules 31-34 are equal to each other and fig. 6 shows the circuit diagram thereof, which can be implemented with electronic components on a board (e.g. PCB type) or in an integrated manner on appropriate media.

Modules 31-34 are electrically fed in direct current via a power supply block 35 connected to the mains power supply at 220 V; preferably, modules 31-34 and the corresponding coils 41-44 are fed in parallel so as to obtain conditions of voltage and/or current substantially equal for all of them.

To this end, the connections of modules 31-34 with the function generator 2 and the power supply block 35 are shown schematically in this sense.

For power control, block 35 is associated with a connection socket 36 with electronic control means (e.g. PLC or the like), not shown in the drawings for simplicity.

The amplifier block 3 is provided with a connection 20 for the connection thereof to the function generator 2, and similar connections 21, 22, 23, 24 for the electrical connection with coils 41, 42, 43, 44; the connections are of the appropriate type depending on the type of signal that is transmitted, power or other, so that sockets, terminals, USB or the like may be used.

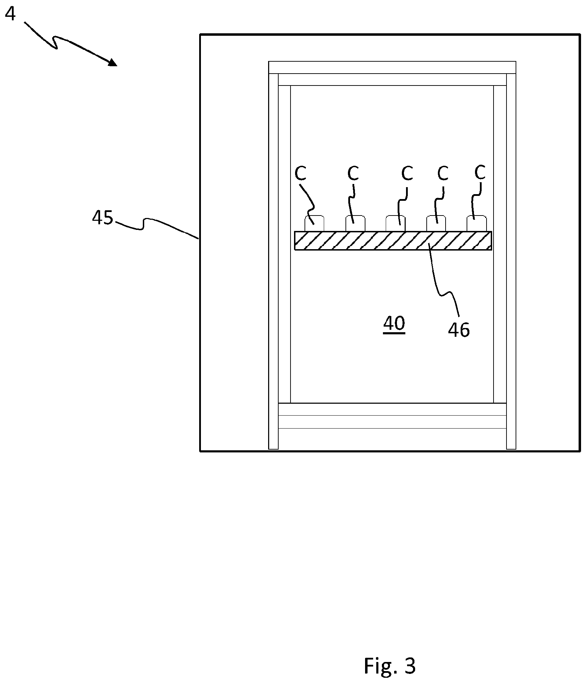

Turning now to consider the magnetic structure 4, as best seen in figures 3-5, in this example it has an essentially parallelepiped geometry, open and/or openable at the ends or on a side, to allow access to its interior by an operator in order to load and unload the cell samples C; it is however to be noted that other shapes of the structure may be adopted, other than the parallelepiped, for example, cylindrical, prismatic, tetrahedral or others.

In particular, cylindrical a configuration or geometry is preferred among the possible alternatives although, in general, the magnetic structure 4 may differ from one case to another in shape and/or size, depending on the applications for which it is intended.

Thus, for example, it may be configured generically as a tunnel which delimits a space in which it is possible to arrange a number of cell samples or specimens C, which may range from a few units to a few tens, depending on the shape and size of the cell samples.

In the magnetic structure 4 is a predetermined area 40, where the magnetic field generated by coils 41-44 is induced; this area is the one where at least one cell sample or specimen C in vitro is arranged (obviously in the case of multiple samples, they will be arranged along the same area 40) for the application of the low-frequency magnetic field, with apparatus 1.

The cell samples are preferably Petri dishes adapted to accommodate a tissue or a culture containing the cells, with a surface area such as to allow the effective application of a magnetic flow according to the needs; the sample plates, which are not shown in the drawings for clarity, are arranged on one or more trays 46 which extend at area 40.

The arrangement of specimens C on a respective tray 46 is adapted to facilitate the linkage of the magnetic flow that traverses them, i.e. with the flow lines are substantially perpendicular to a tissue or cell culture arranged in a specimen.

With reference to the structure or cage 4 in figure 1, the magnetic field lines extend vertically and therefore in the case of a three-dimensional cell culture or tissue, such as for example contained in a test tube or other similar containers, it is supported by tray 46 in upright or vertical condition.

It should however be noted that if one wants to direct the magnetic field only in a particular portion of area 40, for example when a limited number of specimens is used, it is possible to shield the rest of area 40 with shielding means to be applied at the points to be excluded, such as metallic walls or even passive windings.

Coils 41-44 that generate the magnetic field are aligned along a longitudinal axis X-X, which in this case is vertical; however, it should be noted that structure 4 with coils 41-44 and the relative axis X-X may extend horizontally.

Structure 4 preferably has dimensions similar to those of a cabinet or a laboratory equipment in general: the magnetic structure 4 may thus have such dimensions as to be able to install it on a support surface such as a laboratory bench or a doctor's office.

As a function of all these different options, the magnetic structure 4 may be of tubular or box-type, that is to say similar to a tunnel, a cage or a container or otherwise enclosed by a wall or an outer casing 45, and accessible axially or laterally, for example by means of doors, ports and the like.

The tray or trays 46 are preferably configured substantially as drawers extractable and insertable in/from structure 4, which is provided, for this reason, with sliding guides of the trays, not shown in the drawings as they are known per se.

Irrespective of the mentioned configuration, the magnetic structure 4 is provided with magnetic coils 41, 42, 43, 44 arranged side by side along a reference longitudinal direction or axis, whose number and size depend on those of the structure itself; coils 41-44 have the purpose of inducing a uniform magnetic field within the structure or at least a portion thereof.

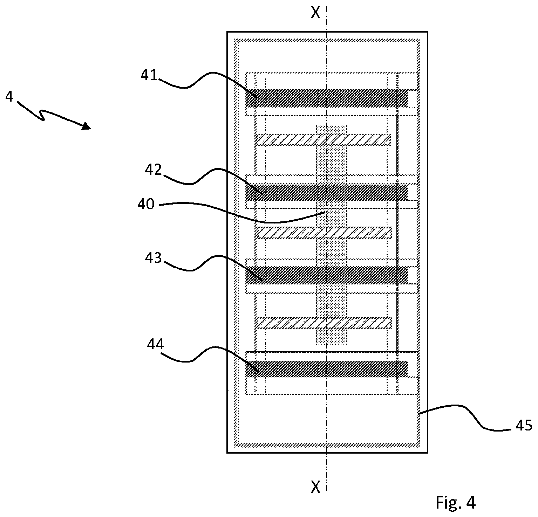

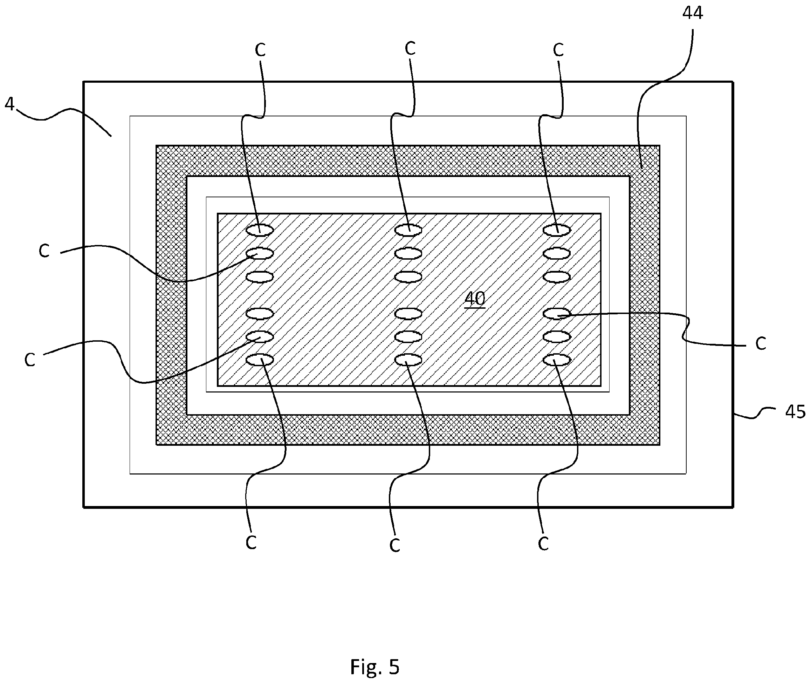

To this end, coils 41-44 are preferably of the so-called "Helmholtz" type, i.e. made with an appropriate number of turns and mutually spaced so as to achieve the desired magnetic field; in the example shown in figures 4 and 5, such a field is represented by the dark area within structure 4.

This is however indicative since the magnetic field area within structure 4 will depend on not only on the configuration thereof (box-like, parallelepiped, cylindrical, etc.), but also on that of coils 41-44 and any other coils or inductors, according to the teaching of the invention.

In fact, according to a preferred embodiment of the latter, the inside of the magnetic structure 4 or at least area 40 where the cell culture or tissue samples to be subjected to the ELF magnetic fields are arranged, is shielded from external environmental magnetic fields, including that of the Earth.

For this purpose, in addition to the vertical-axis Helmoltz coils 41-44 of the example shown, there may be other similar coils oriented differently, such as vertically with respect to the drawings or more generically transverse to the longitudinal axis of the structure, or also directional antennas capable of directing the magnetic field according to the application needs.

The magnetic structure 4 shown in the drawings has an outer height of about 20 cm while that between the outermost coils 41 and 44 is about 16 cm and the pitch between the coils is about 5 cm; advantageously, the coils are not all equal to each other but the outermost ones 41 and 44 have a greater number of turns than the central ones.

For example, one possible solution is to have the outer coils 41, 44 with 180 turns and the inner coils 42, 43 with a number of turns equal to 162; more generally, for a four-coil magnetic structure 4, the ratio of the number of turns of the inner coils to the outer ones may be about 0.9.

Coils 41-44 of structure 4 shown in the drawings are rectangular and have an average height, i.e. referred to the thickness of the windings, of about 10 mm and an average width of 15 cm; structure 4 as a whole is instead about 15 cm large and has a depth of 16 cm.

Of course, these are indicative dimensions that may vary for reasons already explained above, also considering the different shape of structure 4 and of coils 41-44 which may be cylindrical and circular, respectively.

As mentioned, structure 4 is shielded against external magnetic fields, so as to avoid interference in its interior with ELF magnetic fields; the shielding may be of the passive type, i.e. obtained with passive elements such as an outer casing or wall 45, which in a preferred embodiment is made at least partly of ferromagnetic material, so that it can also contain the external flow of coils 41-44.

Shielding from external magnetic interference may also be active, namely obtained with components such as electromagnetic windings external to structure 4.

It is also for this reason that the magnetic structure 4 is preferably located at a certain distance from the amplifier unit 3, in order to avoid electromagnetic interference therewith.

For better control of the operating conditions inside apparatus 1 and, in particular, the magnetic field inside structure 4, it is preferably provided with at least one sensor 47 operatively connected to the function generator 2, which can thus provide to make the necessary corrections to the feeding of coils 41-44 to keep the magnetic field in the desired conditions.

Apparatus 1 further comprises a unit 5 for the detection of cell samples, i.e. for the analysis of those physical magnitudes that allow assessing the effects of applying ELF magnetic fields to the cells.

The detector unit 5 comprises an optical microscope (variable type), adapted for the observation of sample cells subjected to the ELF magnetic fields in structure 4.

According to a possible embodiment, the detection unit 5 further comprises a thermal camera associated with the microscope, so as to also perform measurements of the temperature distribution in the cell samples.

Microscopes suitable for this application are available commercially, such as from the company Olympus Corporation (www.olympus-ims.com) or Bruker Corporation (www.bruker.com).

The information collected by the detector unit 5 are be communicated to a processor unit 6, which comprises an electronic computer or the like and/or other appropriate means (per se known, such as storages, devices, control interface, etc.) to execute an evaluation algorithm and any correction or change of the application process of the magnetic field. To this end, the processor unit 6 is connected to the function generator 2 which therefore receives the feedback data needed to adjust the operating parameters of the magnetic structure 4, such as the frequency of the alternating or pulsed magnetic field, the field strength and/or the power supply current of coils 41-44.

The frequency of the ELF magnetic field generated by coils 41-44 within the magnetic structure 4 is calculated assuming that the main phenomenon that characterizes the cell dissipations and distinguishes the normal cells from cancerous ones is the energy management evaluated as heat exchange with the environment. The assessment of the extent or magnitude of heat exchange is carried out considering that the magnetic field acts on the orientation and/or coupling-uncoupling of the membrane, transmembrane and peripheral protein spins. This effect becomes evident

**EP 3 496 809 B1**

macroscopically with the variation of the surface tension of the membrane and thus with the conditioning of the behavior of molecular motors and channels adapted for the management and realization of transport phenomena. The change of transport phenomena results in a variation of the transmembrane electric potential strength, resulting from a change of electric permittivity (relative dielectric constant).

From the foregoing the operation of apparatus 1 according to the invention can be understood.

In fact, in order to carry out an ELF magnetic field application session, specimens C with the cell samples are placed inside structure 4 at area 40 shown in the figures; the specimens are supported by one or more trays 46 that do not interfere with the magnetic field; to this end, they are preferably made of non-magnetic materials, such as plastic or other non-metallic compounds.

As explained above, within structure 4, coils 41-44 are powered electrically according to the signal produced by generator 2 and amplified by amplifier 3; coils 41-44 can be powered by the sun or, as will be seen in more detail below, in combination with compensation means of external interferences.

In particular, the compensation means are intended to eliminate the external magnetic fields that may interfere with the ELF ones of coils 41-44, such as the Earth's magnetic field or that of equipment or power lines next close to the apparatus.

The compensation means may be of active type, i.e. electrically powered, and in this case they are preferably connected to the amplifier unit 3 which will therefore be different from that shown (as it will have connection to the compensation means), or it may be of the passive type, such as simple short-circuited windings in which the magnetic flow variations induce an electromotive force (according to the well-known Lenz law).

The compensation means may also be implemented in any other appropriate manner, such as a shielding obtained with directional antennas or metal walls and/or baffles.

The magnetic field induced within structure 4 is alternating or pulsed, with low or very low frequency; the latter can range depending on the application, preferably from a few Hz (including fractions such as 0.2-0.5 Hz) up to several tens (90-100 Hz), while the intensity of the magnetic field is preferably between some μT (5-10 μT) and some tens or hundreds μT, typically 100 μT, in addition to a static field of similar magnitude.

The Applicants have found that the application of alternating or pulsed magnetic fields in low or extremely low frequency (ELF or SELF) on tumor cells induces a reduction in proliferation, while this does not happen in healthy cells.

The duration of the application cycles of alternating or pulsed magnetic fields, the frequency and/or intensity thereof depends on various factors such as the type of cells, cell sample sizes (i.e. two-dimensional or three-dimensional); for this reason, at the end of an application cycles the specimens with the cell samples C are removed from structure 4 and analyzed under a microscope 5.

The calculation of frequency $f$ of the magnetic field is obtained by controlling the ratio between the thermal entropy generation rate $S_{g,tf} \approx uL^2\dot{x}_{th}\Delta T\tau_1/6T^2$
and the thermal entropy generation, $S_{g,tf} \approx uL^2\dot{x}_{th}\Delta T\tau_1/6T^2$ i.e.:

$$f = \frac{\dot{S}_{g,tf}}{S_{g,tf}} \approx \frac{hA}{2\rho cV} \qquad \dot{S}_{g,tf} \approx uL^2\dot{x}_{th}\Delta T\big/6T^2 \qquad (1)$$

wherein $L$ is the geometric magnitude characteristic of the convective heat transfer, $\Delta T$ is the temperature difference between the cell and the surrounding environment, $\dot{x}_{th}$ is the thermal stirring rate of the fluid which laps the outer surface of the cell, $T$ is the average temperature of the cell, $u$ is the specific internal energy of the cell, $\tau_1$ is the characteristic time of evolution of the cell thermal process, $\rho$ is the density of the cell, $c$ its specific heat, $V$ its volume, $h$ the convection coefficient and $A$ the area of the outer surface of the cell.

The cell volume is calculated using the geometric characteristics measured using the microscope, while the other biochemical and biophysical properties are taken from literature, for example from the text R. Milo and R. Phillips, Cell Biology by the Numbers, Taylor & Francis, Milton Park, 2015.

The relative value of the amplitude of the magnetic field is obtained considering that the variations in the surface elastic tension are equal to the work of the electric field crossed by the membrane ion flow:

$$\tau \approx qEd_c \qquad\qquad (2)$$

$$B = \sqrt{\mu_m\varepsilon_e}\,E \qquad\qquad (3)$$

wherein $\tau$ is the surface elastic tension of the cell membrane, $q$ is the value of the electric charge, $E$ is the value of the membrane induced electric field (obtained by inverting formula (2)), $d_c$ is the thickness of the membrane, $B$ is the intensity of the magnetic field, $\mu_m$ is the relative magnetic permeability of the membrane and $\varepsilon_e$ is the dielectric constant of the

6

membrane.

Preferably, the results of the processing carried out by the algorithm are obtained essentially in real time, enabling a feedback regulation of the operation of the magnetic structure 4, controlled by the function generator 2.

The pattern of the magnetic field inside structure 4 and other operating parameters of apparatus 1 are preferably displayed with diagrams and/or graphic images.

It is noted that the ability to feedback-change the frequency and/or duration and/or intensity of the alternating or pulsed ELF or SELF electromagnetic fields is allowed or at least favored by the fact that the frequencies of oscillation of magnetic field are low and therefore the image processing time is compatible with that of application of the magnetic fields.

This would not be possible with high frequencies, for example such as those used for MRI scans.

In other words, it can be said that the features of apparatus 1 according to the invention allow making the process of applying alternating or pulsed low frequency electromagnetic fields to the cell samples automatic or semiautomatic.

To this end, the detection unit 5 that comprises the microscope may be integrated with other means (e.g. telecommunication links) so as to automatically provide the data collected to computer 6 which processes them on the basis of the algorithm therein.

These features can thus be exploited for the construction of medical equipment more complex than apparatus 1 considered herein, intended for the treatment also of human or animal body parts.

In general, it can be said that the operating parameters of apparatus 1 depend on several factors: chemical, physical and biological, of the cell samples.

In this case, a very advantageous feature of the invention can be appreciated, that is, to allow having different operating parameters on which to act to provide an optimal treatment, depending on the specific conditions of each cell sample.

In fact, not only the directional magnetic field makes it possible to concentrate the flow locally in the desired points, but also the intensity and the alternating frequency of the field can be adjusted to obtain better results.

It is therefore understandable as these features significantly increase the range of possible applications of the apparatus according to the invention, which can serve not only for mammalian cells but also for those of other organisms.

In fact, several tests have been performed on cell lines in vitro of malignant tumours; among these, the GTL 16, MCF7 and SKBR3 cell lines have shown a slowdown in the proliferation activities.

These results show that low-frequency magnetic fields obtained with the apparatus according to the invention may be a valuable adjuvant treatment of the radiotherapy performed with traditional prior art machines.

Of course, variants of the invention with respect to the example described above are possible, possibly also depending on the various applications.

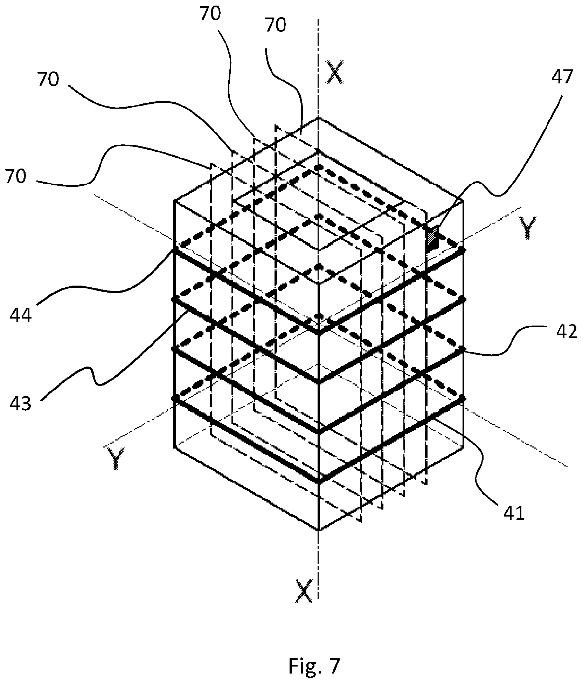

In particular, with reference to figure 7, it shows a magnetic structure 4 comprising in addition to coils 41-44 for generating the low-frequency ELF magnetic field, also compensation and/or protection means against external magnetic interference.

The Applicants have in fact found that the Earth's magnetic field can also, under certain conditions, influence the effects of the ELF magnetic field generated by coils 41-44 on the cells; the compensation or protection means therefore allow overcoming this interference or influences and/or using an additional adjuvant static magnetic field.

To this end, according to a preferred embodiment, the compensation means may be either active, i.e. adapted to generate an autonomous magnetic field which may therefore oppose the external interference of other magnetic fields, such as the Earth's or the like.

Preferably, the compensation means comprise a series of coils 70, 71 with axes respectively perpendicular to the longitudinal one of coils 41-44, so as to act in a plane perpendicular thereto.

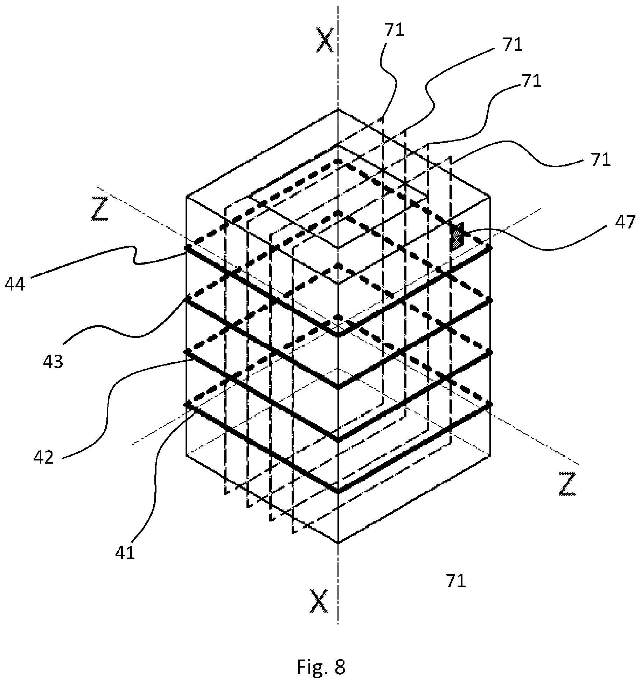

These situations are seen respectively in figures 7 and 8, which show corresponding variants of the magnetic structure 4 where, in the first of them (fig. 7), the compensation coils 70 are aligned along a horizontal axis Y with respect to the longitudinal one X of the structure, while in the second one (fig. 8), the compensation coils are arranged along a horizontal axis Z perpendicular to the other two axes with which it forms a Cartesian tern.

Of course, there may be magnetic structures 4 in which 70 the compensation coils and 71 along axes Y and Z are present simultaneously; moreover, while the compensation coils 70, 71 are preferably of Helmholtz type like those 21-24 that generate the main magnetic field, they may also be of a different type.

In this context, the number and arrangement of the compensation coils 70, 71 may also differ, be larger or smaller, from the four ones shown in the figures.

The compensation coils 70, 71 may also be used for shielding or otherwise modifying the magnetic field at cell specimens which must not be affected by the applications.

The compensation and/or protection coils are preferably 2 fed by the function generator 2 via amplifier 3; advantageously, the field sensor 47 allows detecting the magnetic field variations in the predetermined area 40 within the structure, to provide feedback information that is required for the function generator 2 to operate the compensation and/or protection means 70, 71 so as to compensate for external interferences. The invention is defined by the following claims.

**Claims**

1. Apparatus for influencing cell growth by the application of low-frequency magnetic fields , the apparatus comprising a magnetic structure (4) in which a magnetic field is induced, induction means (2, 3, 41-44) adapted to generate the magnetic field in a predetermined area (40) inside the magnetic structure (4) intended to accommodate at least one cell sample (C), a detection unit (5) for detecting information relating to said at least one in vitro cell sample (C), a processor unit (6) adapted to process said detected information and operatively connected to the induction means (2, 3, 41-44), so as to be able to control the frequency of the magnetic field as a function, at least in part, of the processed information, **characterized in that** the detection unit (5) comprises at least one microscope for observation of the cell samples (C), integrated with means configured to automatically provide the detected information to the processor unit (6).

2. Apparatus according to claims 1, wherein the processor unit (6) is adapted to determine the frequency of the magnetic field generated by the induction means (2, 3, 41-44), as a function of at least one parameter such as the variation of the cell size, or the variation of the total cell mass.

3. Apparatus according to any one of the preceding claims, comprising compensation means (45, 70, 71) for substantially shielding said predetermined area (40) of the magnetic field within the structure from external magnetic field interferences.

4. Apparatus according to claim 3, wherein said compensation means (45, 70, 71) comprise at least one coil (70, 71) connected to the induction means (2, 3, 41-44) of the magnetic field within the structure (4).

5. Apparatus according to any one of the preceding claims, wherein the induction means (2, 3, 41-44) and/or the compensation means (45, 70, 71) of the magnetic field comprise Helmholtz coils and/or directional antennas.

6. Apparatus according to any one of the preceding claims, wherein the magnetic field frequencies are in the range of between 0.2 and 200 Hz.

7. Apparatus according to any one of the preceding claims, wherein the magnetic structure (4) has a substantially box-like or tunnel configuration.

8. Apparatus according to any one of the preceding claims, wherein the magnetic structure (4) comprises a plurality of coils (41-44) arranged side by side along a reference direction, and wherein the outermost coils (41, 44) have a number of turns greater than the innermost ones (42, 43).

9. Apparatus according to any one of the preceding claims, comprising means (2, 3, 6) for varying the frequency and/or intensity of the magnetic field as a function of chemical and/or physical and/or biological characteristics of tumor cells to be treated.

10. Apparatus according to any one of the preceding claims, wherein the detection unit (5) further comprises a thermal camera associated with the microscope, so as to also perform measurements of the temperature distribution in the cell samples, and the frequency $f$ of the magnetic field is controlled as a function of the ratio between the thermal entropy generation rate $\dot{S}_{g,tf} \approx uL^2\dot{x}_{th}\Delta T/6T^2$ and the thermal entropy generation $S_{g,tf} \approx uL^2\dot{x}_{th}\Delta T\tau_1/6T^2$, i.e.:

$$f = \frac{\dot{S}_{g,tf}}{S_{g,tf}} \approx \frac{hA}{2\rho cV} \qquad (1)$$

wherein $L$ is the geometric magnitude characteristic of the convective heat transfer, $\Delta T$ is the temperature difference between the cell and the surrounding environment , $\dot{x}_{th}$ is the thermal stirring rate of the fluid which laps the outer surface of the cell, $T$ is the average temperature of the cell, $u$ is the specific internal energy of the cell, $\tau_1$ is the characteristic time of evolution of the cell thermal process, $\rho$ is the density of the cell, $c$ its specific heat, $V$ its volume, $h$ the convection coefficient and $A$ the area of the outer surface of the cell.

11. Apparatus according to any one of the preceding claims, wherein the amplitude of the magnetic field is controlled considering that the variations in the surface elastic tension are equal to the work of the electric field crossed by the

membrane ion flow:

$$\tau \approx qEd_c \qquad (2)$$

$$B = \sqrt{\mu_m \varepsilon_e}\, E \qquad (3)$$

wherein $\tau$ is the surface elastic tension of the cell membrane, $q$ is the value of the electric charge, $E$ is the value of the membrane induced electric field, $d_c$ is the thickness of the membrane, $B$ is the intensity of the magnetic field, $\mu_m$ is the relative magnetic permeability of the membrane and $\varepsilon_e$ is the dielectric constant of the membrane.

12. Method for influencing the in vitro cell growth by the application of low-frequency magnetic fields with an apparatus according to any of the preceding claims, wherein the detection unit further comprises a thermal camera associated with the microscope, so as to also perform measurements of the temperature distribution in the cell samples, **characterized in that** the frequency $f$ of the magnetic field is controlled as a function of the ratio between the thermal entropy generation rate $\dot{S}_{g,tf} \approx uL^2\dot{x}_{th}\Delta T/6T^2$ and the thermal entropy generation $S_{g,tf} \approx uL^2\dot{x}_{th}\Delta T\tau_1/6T^2$, i.e.:

$$f = \frac{S_{g,tf}}{S_{g,tf}} \approx \frac{hA}{2\rho cV} \qquad (1)$$

wherein $L$ is the geometric magnitude characteristic of the convective heat transfer, $\Delta T$ is the temperature difference between the cell and the surrounding environment , $\dot{x}_{th}$ is the thermal stirring rate of the fluid which laps the outer surface of the cell, $T$ is the average temperature of the cell, $u$ is the specific internal energy of the cell, $\tau_1$ is the characteristic time of evolution of the cell thermal process, $\rho$ is the density of the cell, $c$ its specific heat, V its volume, $h$ the convection coefficient and $A$ the area of the outer surface of the cell.

13. Method according to claim 12, wherein the amplitude of the magnetic field is controlled considering that the variations in the surface elastic tension are equal to the work of the electric field crossed by the membrane ion flow:

$$\tau \approx qEd_c \qquad (2)$$

$$B = \sqrt{\mu_m \varepsilon_e}\, E \qquad (3)$$

wherein $\tau$ is the surface elastic tension of the cell membrane, $q$ is the value of the electric charge, $E$ is the value of the membrane induced electric field, $d_c$ is the thickness of the membrane, $B$ is the intensity of the magnetic field, $\mu_m$ is the relative magnetic permeability of the membrane and $\varepsilon_e$ is the dielectric constant of the membrane.

**Patentansprüche**

1. Vorrichtung zur Beeinflussung des Zellwachstums durch Anwenden niederfrequenter Magnetfelder, wobei die Vorrichtung eine Magnetstruktur (4) umfasst, in der ein Magnetfeld induziert wird, Induktionsmittel (2, 3, 41-44) zum Erzeugen des Magnetfeldes in einem vorbestimmten Bereich (40) innerhalb der Magnetstruktur (4), der zur Aufnahme mindestens einer Zellprobe (C) bestimmt ist, eine Detektionseinheit (5) zum Erfassen von Informationen, die sich auf die mindestens eine in vitro Zellprobe (C) beziehen, eine Prozessoreinheit (6) zum Verarbeiten der erfassten Informationen, die mit den Induktionsmitteln (2, 3, 41-44) verbunden ist, um die Frequenz des Magnetfeldes zumindest teilweise in Abhängigkeit von den verarbeiteten Informationen steuern zu können, **dadurch gekennzeichnet, dass** die Detektionseinheit (5) mindestens ein Mikroskop zur Beobachtung der Zellproben (C) umfasst, das mit Mitteln integriert ist, die so konfiguriert sind, dass sie die erfassten Informationen automatisch an die Prozessoreinheit (6) liefern.

2. Vorrichtung nach Anspruch 1, wobei die Prozessoreinheit (6) angepasst ist, die Frequenz des von den Induktionsmitteln (2, 3, 41-44) erzeugten Magnetfelds in Abhängigkeit von mindestens einem Parameter, wie der Änderung der Zellgröße oder der Änderung der Gesamtzellmasse, zu bestimmen.

3. Vorrichtung nach einem der vorstehenden Ansprüche, mit Kompensationsmitteln (45, 70, 71) zum weitgehenden Abschirmen des vorbestimmten Bereichs (40) des Magnetfelds innerhalb der Struktur von externen Magnetfeldstörungen.

4. Vorrichtung nach Anspruch 3, wobei die Kompensationsmittel (45, 70, 71) mindestens eine Spule (70, 71) umfassen, die mit den Induktionsmitteln (2, 3, 41-44) des Magnetfelds innerhalb der Struktur (4) verbunden ist.

5. Vorrichtung nach einem der vorstehenden Ansprüche, wobei die Induktionsmittel (2, 3, 41-44) und/oder die Kompensationsmittel (45, 70, 71) des Magnetfeldes Helmholtz-Spulen und/oder Richtantennen umfassen.

6. Vorrichtung nach einem der vorstehenden Ansprüche, wobei die Magnetfeldfrequenzen im Bereich zwischen 0,2 und 200 Hz liegen.

7. Vorrichtung nach einem der vorstehenden Ansprüche, wobei die Magnetstruktur (4) eine im Wesentlichen kastenförmige oder tunnelartige Konfiguration aufweist.

8. Vorrichtung nach einem der vorstehenden Ansprüche, wobei die Magnetstruktur (4) mehrere Spulen (41-44) umfasst, die nebeneinander entlang einer Referenzrichtung angeordnet sind, und wobei die äußersten Spulen (41, 44) eine größere Anzahl von Windungen aufweisen als die innersten (42, 43).

9. Vorrichtung nach einem der vorstehenden Ansprüche, mit Mitteln (2, 3, 6) zum Variieren der Frequenz und/oder Intensität des Magnetfeldes in Abhängigkeit von chemischen und/oder physikalischen und/oder biologischen Eigenschaften von zu behandelnden Tumorzellen.

10. Vorrichtung nach einem der vorstehenden Ansprüche, wobei die Detektionseinheit (5) ferner eine mit dem Mikroskop verbundene Wärmebildkamera umfasst, um auch Messungen der Temperaturverteilung in den Zellproben durchzuführen, und die Frequenz $f$ des Magnetfelds in Abhängigkeit vom Verhältnis zwischen der thermischen Entropieerzeugungsrate $\dot{S}_{g,tf} \approx uL^2\dot{x}_{th}\Delta T/6T^2$ und der thermischen Entropieerzeugung $S_{g,tf} \approx uL^2\dot{x}_{th}\Delta T\tau_1$/gesteuert wird, d. h.:

$$f = \frac{\dot{S}_{g,tf}}{S_{g,tf}} \approx \frac{hA}{2pcV} \qquad (1)$$

wobei $L$ die geometrische Größe ist, die für die konvektive Wärmeübertragung charakteristisch ist, $\Delta T$ die Temperaturdifferenz zwischen der Zelle und der Umgebung ist, $\dot{x}_{th}$ die thermische Rührgeschwindigkeit der Flüssigkeit ist, die die Außenfläche der Zelle umspült, $T$ die durchschnittliche Temperatur der Zelle ist, u die spezifische innere Energie der Zelle ist, $\tau_1$ die charakteristische Zeit der Entwicklung des thermischen Prozesses der Zelle ist, $p$ die Dichte der Zelle ist, $c$ ihre spezifische Wärme ist, $V$ ihr Volumen ist, $h$ der Konvektionskoeffizient ist und A die Fläche der Außenfläche der Zelle ist.

11. Vorrichtung nach einem der vorstehenden Ansprüche, wobei die Amplitude des Magnetfelds unter Berücksichtigung der Tatsache gesteuert wird, dass die Schwankungen der Oberflächenelastizität gleich der Arbeit des elektrischen Feldes sind, das von dem Membranionenfluss durchquert wird:

$$\tau \approx qEd_c \qquad (2)$$

$$B = \sqrt{\mu_m\varepsilon_e E} \qquad (3)$$

wobei $\tau$ die Oberflächenelastizität der Zellmembran ist, $q$ der Wert der elektrischen Ladung ist, $E$ der Wert des durch die Membran induzierten elektrischen Feldes ist, $d_c$ die Dicke der Membran ist, $B$ die Intensität des Magnetfeldes ist, $\mu_m$ die relative magnetische Permeabilität der Membran ist und $\varepsilon_e$ die Dielektrizitätskonstante der Membran ist.

12. Verfahren zur Beeinflussung des in vitro Zellwachstums durch Anwendung niederfrequenter Magnetfelder mit einer Vorrichtung nach einem der vorstehenden Ansprüche, wobei die Detektionseinheit ferner eine mit dem Mikroskop verbundene Wärmebildkamera umfasst, um auch Messungen der Temperaturverteilung in den Zellproben durchzuführen, **dadurch gekennzeichnet, dass** die Frequenz f des Magnetfeldes in Abhängigkeit vom Verhältnis zwischen der thermischen Entropieerzeugungsrate $\dot{S}_{g,tf} \approx uL^2\dot{x}_{th}\Delta T/6T^2$ und der thermischen Entropieerzeugung $S_{g,tf} \approx uL^2\dot{x}_{th}\Delta T\tau_1/6T^2$ gesteuert wird, d. h.:

$$f = \frac{\dot{S}_{g,tf}}{S_{g,tf}} \approx \frac{hA}{2pcV} \qquad (1)$$

wobei $L$ die geometrische Größe ist, die für die konvektive Wärmeübertragung charakteristisch ist, $\Delta T$ die Temperatur-differenz zwischen der Zelle und der Umgebung ist, $\dot{x}_{th}$ die thermische Rührgeschwindigkeit der Flüssigkeit ist, die die Außenfläche der Zelle umspült, $T$ die durchschnittliche Temperatur der Zelle ist, $u$ die spezifische innere Energie der Zelle ist, $\tau_1$ die charakteristische Zeit der Entwicklung des thermischen Prozesses der Zelle ist, $p$ die Dichte der Zelle ist, $c$ ihre spezifische Wärme ist, $V$ ihr Volumen ist, $h$ der Konvektionskoeffizient ist und $A$ die Fläche der Außenfläche der Zelle ist.

**13.** Verfahren nach Anspruch 12, wobei die Amplitude des Magnetfelds unter Berücksichtigung der Tatsache gesteuert wird, dass die Schwankungen der Oberflächenelastizität gleich der Arbeit des elektrischen Feldes sind, das vom Membranionenfluss durchquert wird:

$$\tau \approx qEd_c \qquad (2)$$

$$B = \sqrt{\mu_m \varepsilon_e E} \qquad (3)$$

wobei $\tau$ die Oberflächenelastizität der Zellmembran ist, $q$ der Wert der elektrischen Ladung ist, $E$ der Wert des durch die Membran induzierten elektrischen Feldes ist, $d_c$ die Dicke der Membran ist, $B$ die Intensität des Magnetfeldes ist, $\mu_m$ die relative magnetische Permeabilität der Membran ist und $\varepsilon_e$ die Dielektrizitätskonstante der Membran ist.

## Revendications

**1.** - Appareil pour influencer une croissance cellulaire par l'application de champs magnétiques à basse fréquence, l'appareil comprenant une structure magnétique (4) dans laquelle un champ magnétique est induit, des moyens d'induction (2, 3, 41-44) aptes à générer le champ magnétique dans une zone prédéterminée (40) à l'intérieur de la structure magnétique (4) destinée à recevoir au moins un échantillon cellulaire (C), une unité de détection (5) pour détecter des informations se rapportant audit au moins un échantillon cellulaire in vitro (C), une unité de processeur (6) apte à traiter lesdites informations détectées et connectée de manière fonctionnelle aux moyens d'induction (2, 3, 41-44) de façon à être apte à commander la fréquence du champ magnétique en fonction, au moins en partie, des informations traitées, **caractérisé par le fait que** l'unité de détection (5) comprend au moins un microscope pour l'observation des échantillons cellulaires (C), intégré à des moyens configurés pour fournir automatiquement les informations détectées à l'unité de processeur (6).

**2.** - Appareil selon la revendication 1, dans lequel l'unité de processeur (6) est apte à déterminer la fréquence du champ magnétique généré par les moyens d'induction (2, 3, 41-44), en fonction d'au moins un paramètre tel que la variation de la taille de cellule, ou la variation de la masse cellulaire totale.

**3.** - Appareil selon l'une quelconque des revendications précédentes, comprenant des moyens de compensation (45, 70, 71) pour protéger substantiellement ladite zone prédéterminée (40) du champ magnétique à l'intérieur de la structure vis-à-vis d'interférences de champs magnétiques externes.

**4.** - Appareil selon la revendication 3, dans lequel lesdits moyens de compensation (45, 70, 71) comprennent au moins une bobine (70, 71) connectée aux moyens d'induction (2, 3, 41-44) du champ magnétique à l'intérieur de la structure (4).

**5.** - Appareil selon l'une quelconque des revendications précédentes, dans lequel les moyens d'induction (2, 3, 41-44) et/ou les moyens de compensation (45, 70, 71) du champ magnétique comprennent des bobines de Helmholtz et/ou des antennes directionnelles.

**6.** - Appareil selon l'une quelconque des revendications précédentes, dans lequel les fréquences de champ magnétique sont dans la plage comprise entre 0,2 et 200 Hz.

**7.** - Appareil selon l'une quelconque des revendications précédentes, dans lequel la structure magnétique (4) a une configuration sensiblement en forme de boîte ou de tunnel.

**8.** - Appareil selon l'une quelconque des revendications précédentes, dans lequel la structure magnétique (4) comprend une pluralité de bobines (41-44) disposées côte à côte le long d'une direction de référence, et dans lequel les bobines les plus à l'extérieur (41, 44) ont un nombre de spires supérieur à celui des bobines les plus à l'intérieur (42, 43).

**9.** - Appareil selon l'une quelconque des revendications précédentes, comprenant des moyens (2, 3, 6) pour faire varier la fréquence et/ou l'intensité du champ magnétique en fonction de caractéristiques chimiques et/ou physiques et/ou biologiques de cellules tumorales à traiter.

**10.** - Appareil selon l'une quelconque des revendications précédentes, dans lequel l'unité de détection (5) comprend en outre une caméra thermique associée au microscope, de façon à réaliser également des mesures de la répartition de température dans les échantillons cellulaires, et la fréquence f du champ magnétique est commandée en fonction du rapport entre la vitesse de génération d'entropie thermique $\dot{S}_{g,tf} \approx uL^2\dot{x}_{th}\Delta T/6T^2$ et la génération d'entropie thermique $S_{g,tf} \approx uL^2\dot{x}_{th}\Delta T\tau_1/6T^2$, à savoir :

$$f = \frac{\dot{S}_{g,tf}}{S_{\sigma,tf}} \approx \frac{hA}{2\rho c V} \qquad (1)$$

où L est la caractéristique de grandeur géométrique du transfert de chaleur par convection, $\Delta T$ est la différence de température entre la cellule et le milieu environnant, $\dot{x}_{th}$ est la vitesse d'agitation thermique du fluide qui tapisse la surface externe de la cellule, $T$ est la température moyenne de la cellule, $u$ est l'énergie interne spécifique de la cellule, $\tau_1$ est le temps caractéristique d'évolution du processus thermique de la cellule, $p$ est la densité de la cellule, $c$ sa chaleur spécifique, $V$ son volume, $h$ le coefficient de convection et $A$ l'aire de la surface externe de la cellule.

**11.** - Appareil selon l'une quelconque des revendications précédentes, dans lequel l'amplitude du champ magnétique est commandée en considérant que les variations de la tension élastique de surface sont égales au travail du champ électrique traversé par le flux ionique membranaire :

$$\tau \approx qEd_c \qquad (2)$$

$$B = \sqrt{\mu_m \varepsilon_e} E \qquad (3)$$

où $\tau$ est la tension élastique de surface de la membrane cellulaire, $q$ est la valeur de la charge électrique, $E$ est la valeur du champ électrique induit par la membrane, $d_c$ est l'épaisseur de la membrane, $B$ est l'intensité du champ magnétique, $\mu_m$ est la perméabilité magnétique relative de la membrane et $\varepsilon_e$, est la constante diélectrique de la membrane.

**12.** - Procédé pour influencer la croissance cellulaire in vitro par l'application de champs magnétiques à basse fréquence avec un appareil selon l'une quelconque des revendications précédentes, dans lequel l'unité de détection comprend en outre une caméra thermique associée au microscope, de façon à effectuer également des mesures de la répartition de température dans les échantillons cellulaires, **caractérisé par le fait que** la fréquence $f$ du champ magnétique est commandée en fonction du rapport entre la vitesse de génération d'entropie thermique $\dot{S}_{g,tf} \approx uL^2\dot{x}_{th}\Delta T/6T^2$ et la génération d'entropie thermique $S_{g,tf} \approx uL^2\dot{x}_{th}\Delta T\tau_1/6T^2$, à savoir :

$$f = \frac{S_{g,tf}}{S_{\sigma,tf}} \approx \frac{hA}{2\rho c V} \qquad (1)$$

où *L est* la caractéristique de grandeur géométrique du transfert thermique par convection, $\Delta T$ est la différence de température entre la cellule et le milieu environnant, $\dot{x}_{th}$ est la vitesse d'agitation thermique du fluide qui tapisse la surface externe de la cellule, *T* est la température moyenne de la cellule, u est l'énergie interne spécifique de la cellule, $\tau_1$ est le temps caractéristique d'évolution du processus thermique de la cellule, *p* est la densité de la cellule, *c* sa chaleur spécifique, *V* son volume, *h* le coefficient de convection et A l'aire de la surface externe de la cellule.

13. - Procédé selon la revendication 12, dans lequel l'amplitude du champ magnétique est commandée en considérant que les variations de la tension élastique de surface sont égales au travail du champ électrique traversé par le flux ionique membranaire :

$$\tau \approx qEd_c \qquad\qquad (2)$$

$$B = \sqrt{\mu_m \varepsilon_e} \, E \qquad\qquad (3)$$

où $\tau$ est la tension élastique de surface de la membrane cellulaire, *q* est la valeur de la charge électrique, *E* est la valeur du champ électrique induit par la membrane, $d_c$ est l'épaisseur de la membrane, *B* est l'intensité du champ magnétique, $\mu_m$ est la perméabilité magnétique relative de la membrane et $\varepsilon_e$ est la constante diélectrique de la membrane.

Fig. 1

Fig. 2

Fig. 3

Fig. 4

Fig. 5

Fig. 6

Fig. 7

EP 3 496 809 B1

Fig. 8

EP 3 496 809 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013048546 A1
- WO 2005010162 A2
- WO 2009153818 A1
- EP 0966988 A

### Non-patent literature cited in the description

- **H. BERG** ; **B. GUNTHER** ; **I. HILGER** ; **M. RADEVA** ; **N. TRAITCHEVA** ; **L. WOLLBER**. Bioelectromagnetic Field Effects on Cancer Cells and Mice Tumors. *Electromagnetic Biology and Medicine*, 2010, vol. 29, 132-143
- **J. SCHUDERER** ; **W. OESCH** ; **N. FELBER** ; **D. SPÄT** ; **N. KUSTER**. In Vitro Exposure Apparatus for ELF Magnetic Fields. *Bioelectro magnetics*, 2004, vol. 25, 582-591